# EUROPEAN PATENT APPLICATION

(11) **EP 4 509 058 A1**
(43) Date of publication of application: **19.02.2025**
(21) Application number: 23191872.3
(22) Date of filing: 17.08.2023
(51) Int. Cl.: A61B 6/06

(54) **APPARATUS FOR COLLIMATING X-RAY BEAMS**

(71) Applicant: Koninklijke Philips N.V., 5656 AG Eindhoven (NL)
(72) Inventor: LUNDT, Bernd, Eindhoven (NL); KOEPNICK, Johannes, Eindhoven (NL); MAY, Jan Marek, Eindhoven (NL)
(74) Representative: Philips Intellectual Property & Standards

(57) **Abstract**

The invention concerns an apparatus comprising a collimator configured for collimate electromagnetic radiation emitted from an X-ray source, a sensor configured for acquiring data of an anatomy of a patient, and a processing unit. The processing unit is configured to calculate an optimal collimation position of the collimator based on the acquired data of the sensor, determine a movement of the collimator based on the calculated optimal collimation position, wherein the movement of the collimator comprises a rotation of the collimator around a central axis of the collimator. The invention further concerns an X-ray system comprising the apparatus and a method for moving a collimator for collimate electromagnetic radiation emitted form an X-ray source.

## Description

### FIELD OF THE INVENTION

The present invention relates to the field of radiation based imaging. In particular the invention relates to the field of X-ray collimation, in particular to a collimator for collimating an X-ray beam and a system, an apparatus comprising such collimator, and to a method for collimating an X-ray beam.

### BACKGROUND OF THE INVENTION

Appropriate X-ray beam collimation is important when using an X-ray source. The collimation of the X-ray beam reduces the amount of irradiation/ ionizing radiation to which a patient is exposed und thus minimizes radiation risks. Furthermore, appropriate collimation reduces the amount of scattered radiation since less volume is irradiated which results in an improved detail contrast and image quality.

For many examinations like chest lateral and orthopedic examinations it is often necessary to manually rotate the collimator for reducing the unnecessarily irradiated area. This is done by the medical personnel by manually rotating the collimator. The collimation workflow requires a medical person, a lab technician, to introduce and to position the patient to the image acquisition, to adjust detector and X-ray tube and to adjust collimator settings. This setting and arrangement requires much time for an imaging session, which is taken up by appropriate patient and system positioning including collimation.

### SUMMARY OF THE INVENTION

There may therefore be a need for an apparatus to ease the handling and arrangement of the collimation process for medical personnel either in preparation of or during an image acquisition session or run. Further, there may therefore be a need for an apparatus which improves the imaging process based on the collimation process.

An object of the invention is to provide an apparatus, a system and a method for an optimal collimation, wherein the irradiation of the patient during an imaging process is reduced and the workload can be eased and reduced.

The object of the present invention is solved by the subject matter of the independent claims where further embodiments are incorporated in the dependent claims. It is understood that the following aspect of the invention equally applies to the method, the X-ray system, to the computer program element and to the computer readable medium. Accordingly, any feature, function, step and/or element described in the following with reference to one aspect of the present disclosure equally applies to any other aspect of the present disclosure.

According to a first aspect of the invention there is provided an apparatus comprising a collimator configured for collimate electromagnetic radiation emitted from an X-ray source, a sensor configured for acquiring data of an anatomy of a patient, and a processing unit. The processing unit is configured to calculate an optimal collimation position of the collimator based on the acquired data of the sensor, determine a movement of the collimator based on the calculated optimal collimation position, wherein the movement of the collimator comprises a rotation of the collimator around a central axis of the collimator.

In the context of the present invention, the term "optimal collimation position" shall be understood to describe a position of the collimator, wherein the irradiated area of the patient is minimal in view of the area which have to be irradiated for a significant and meaningful image of the anatomy of the patient. On the other side the area for a significant image has to be large enough. In other words, the optimal collimation position is a position of the collimator and its elements in relation to a collimation area which should be as small as possible but should also be large enough for a sufficient image for X-ray acquisition. Therefore, depending on the object, the anatomy of the patient, to be examined, and taking into account the medical and/or clinical guidelines for examination of respective parts of a patient the optimal collimation position may differ and along with that the irradiated area may be different.

In the context of the present invention, the term "anatomy of a patient" shall be understood to describe at least a part of a patient which should undergo an image acquisition, hence which should undergo an examination procedure. For example, the anatomy of a patient may be any body part like a hand, a chest, a leg or other.

In the context of the present invention, the term "movement of the collimator" shall be understood to describe the aligning and/or adjusting of the collimator or parts of the collimator. For instance the whole collimator and/or parts of the collimator are moved for achieving the optimal collimation position. In particular the movement of the collimator relates to a rotation of the collimator.

In the context of the present invention, the term "central axis of the collimator" shall be understood to describe a rotation axis of the collimator. The central axis may be perpendicular to the X axis and the Y axis. When viewed from the direction of the electromagnetic radiation, which is emitted from the X-ray source to the direction of the collimator and further on the anatomy of the patient, the central axis of the collimator may be parallel to the imaging direction of the electromagnetic radiation. In particular the central axis of the collimator may be identical with the extending direction of a central beam of the X-ray source, which extends into the direction of the anatomy of the patient from the X-ray source.

In other words, the processor may be configured for controlling and/or setting movement of the collimator but may be not moving the collimator. The medical personnel may use the information about the optimal collimation position depending on the examination to be performed and the movement of the collimator. The rotation, may be done manually by the medical personal but knowing the optimal collimation position which could be set properly.

The processor may further be configured to provide the calculated optimal collimation position for further processing units and/or steps, as for example described with the further embodiments herein. The processor unit may use software which calculates the collimation based on the acquired data. The collimator which is configured for collimate electromagnetic radiation emitted from the X-ray source is in other words configured for generating an collimation area which reduces the area irradiated by the electromagnetic radiation emitted from the X-ray source at the examined anatomy of the patient. The irrigated area should be as small as possible which is achieved by the collimator having the optimal collimation position. The apparatus is a single element and may be in communication or in contact with the X-ray source, wherein the X-ray source must not be part of the apparatus, hence may be an external X-ray source.

With an apparatus configured in such a manner as described with the embodiments herein the collimation of the collimator can be improved, in particular the optimized collimation position for the collimator can be defined and used such that the irradiated area for the patient and therefore the amount of X-ray radiation transmitted to the patient can be reduced. This leads to an improvement of the patient safety during X-ray examination and reduces the workload of the medical personal during X-ray imaging/acquisitions. Due to the rotation of the collimator the additional degree of freedom, of the rotation, can be taken into account and the optimal collimation can be calculated. Especially for unexperienced radiographers the apparatus helps to adjust the best collimation position without knowing the best point for reducing the irradiated area.

According to an exemplary embodiment of the invention, the sensor may be a camera and/or the sensor may be any device for receiving the data about the anatomy of the patient. For instance possible sensors may be infrared cameras, US camera, or line sensors. The acquired data may be at least one picture of an anatomy of a patient which may be further analyzed by the processing unit for calculating an optimal collimation position of the collimator.

According to an exemplary embodiment of the invention, the processing unit may be configured to control the collimator based on the calculated optimal collimation position. In particular the movement of the collimator, the rotation of the collimator may be controlled using the processing unit based on the calculated optimal collimation position. For instance the processing unit may indicate a start and/or stop position of the collimator for rotating into the respective direction. Further, the processing unit may control how the information about the optimal collimation position is transmitted to a further elements/devices for moving the collimator.

According to an exemplary embodiment of the invention, the calculation of the optimal collimation position may comprise calculating a rotation angle for rotating the collimator around the central axis of the collimator. For instance the collimator may be rotatable forward and backward around the central axis of the collimator. The rotating angle may be defined as the angle of rotation between an X axis and the Y axis, to which the central axis of the collimator is perpendicular.

According to an exemplary embodiment of the invention, the apparatus may further comprise a rotatable mounting element, wherein the collimator may be attached to the rotatable mounting element, which may be configured for rotating the collimator around the central axis of the collimator. The apparatus may further comprise a moving unit, wherein the mounting element is rotatable by the moving unit. The mounting element may be moved independently of the moving unit, wherein the moving unit is only used for moving the collimator. On the other hand the moving unit may move the mounting element together with the collimator. The moving unit may be a motorized mechanic which is able to rotate the collimator and its components. The application of software for moving the moving unit may be possible, wherein the software may be executed by the processing unit, such that the processing unit may control the moving unit, hence the motorized mechanic.

According to an exemplary embodiment of the invention, the processing unit may be further configured to control the moving unit depending on the calculated optimal collimation position for rotating the collimator. For instance the processing unit may only be configured to control the moving unit and not the collimator for moving the collimator. On the other hand the processing unit may control both the moving unit and the collimator for moving, i.e. rotating, the collimator. The controlling may comprise the providing of the information about the optimal collimation position to the moving unit such that the moving unit (and/or the collimator) could be moved by further elements to the optimal collimation position.

According to an exemplary embodiment of the invention, the processing unit may be further configured to calculate a first movement of the collimator along a first direction, and calculate a second movement of the collimator along a second direction, wherein the first direction and the second direction are perpendicular to each other. In particular, the first direction may be the X-axis direction of the collimator and the second direction may be the Y-axis direction of the collimator, wherein this is illustrated in detail in the embodiments described with the Figures. The calculating of the first movement and second movement may be independently from each other or may be performed simultaneously. Further, the calculation of the first and second movement may be independent steps or may be carried out by the processing unit in combination.

According to an exemplary embodiment of the invention, the processing unit may be further configured to use the position of an X-ray detector for improving the calculation of the optimal collimation position and/or to move the X-ray detector, wherein the processing unit is in connection and in communication with an X-ray system comprising the X-ray detector. Furthermore, the processing unit may also be configured to move an X-ray source depending on the calculated optimal collimation position for improving the collimation, wherein the X-ray source is a part of the X-ray system with which the processing unit is in connection and in communication. When moving the X-ray source and/or the detector further motorized mechanics may be used for each extra device which movement should be controlled by the processing unit.

According to an exemplary embodiment of the invention, the processing unit may be further configured to control the optimal collimation position of the collimator in the first direction and/or in the second direction, wherein the collimator may be moved to the optimal collimation position based on at least one of the calculated first movement, second movement, or the rotation of the collimator. The processing unit may be able to control the movement in the first and/or second direction, wherein each direction may be controlled separable, for instance when the collimator is only controlled in one direction, or the directions may be controlled simultaneously. For example the collimator may only be moved into one direction such that the movement to the other direction or the rotation may be omitted or the collimator may only be rotated such that a movement into the first and/or second direction may be omitted. On the other hand the collimator may be moved to the optimal collimation position by moving along the first direction, along the second direction and by being rotated.

According to an exemplary embodiment of the invention, wherein the processing unit may be further configured to calculate the rotation of the collimator simultaneously with at least one of the calculation of the movement of the collimator along the first direction or the calculation of the movement of the collimator along the second direction. Accordingly, the processing unit may not only be configured to calculate the movement into the different directions and the rotation independently from each other but also to calculate them simultaneously.

According to an exemplary embodiment of the invention, the collimator may comprise movable collimator plates configured for being moved along the first direction and/or along the second direction for forming a collimation area. In particular, the collimator may comprise at least four movable collimator plates. The processing unit may be configured to control the optimal collimator position by controlling the position of the collimator plates. For instance the processing unit may be configured to control each collimator plate individually and/or the processing unit may be configured to control more than one collimator plate simultaneously. In particular respective pairs of collimator plates may be controlled together by the processing unit. Accordingly the processing unit may be configured to move one or more collimator plates along the first direction and/or along the second direction. As described hereinabove with the embodiment of the whole collimator, also the plates of the collimator may be moved to the optimal collimation position based on at least one of the calculated first movement, second movement or the rotation of the collimator.

Further, the individually collimator plates may be movable along the first direction and/or the second direction, wherein the first direction may be an X-axis direction and wherein the second direction may be a Y-axis direction.

According to an exemplary embodiment of the invention, the apparatus may further comprise a housing, wherein the collimator is arranged inside the housing and wherein the collimator is rotatable inside the housing. In other words the collimator is rotatably arranged inside the housing. For instance the collimator mounted on the mounting element is arranged inside the housing. Further, the moving unit may be arranged inside the housing. Hence, it may be possible to arrange all components which should move the collimator to the optimal collimation position inside the housing. The housing may be an additional housing of the collimator and may be different than a housing of an X-ray source.

According to an exemplary embodiment of the invention, the processing unit may be further configured to determine anatomical landmarks from the acquired data, wherein the optimal collimation is calculated based on/using the determined anatomical landmarks. For instance the anatomical landmarks may be acquired from image data. The optimal collimation may be calculated using the position of these landmarks. The anatomical landmarks may be predefined landmarks which depend on the respective anatomy of the patient.

According to an exemplary embodiment of the invention, the processing unit may be further configured to calculate the optimal collimation based on at least one of a trained neural network using end-to end learning, or deep learning. The neural network may be trained based on landmarks acquired from data, for example image data, wherein the landmarks may be acquired from different body parts, which means difference anatomies of a patient. For instance the neural network may be trained using previously acquired landmarks of different patients and or/of different anatomies of these patients.

According to an exemplary embodiment of the invention, the apparatus may further comprise a user interface configured to display to a user the calculated optimal collimation and/or wherein the processor may be further configured to provide a guidance to the user for receiving optimal collimation. In particular the guidance may be provided independently from the displaying of the information to the user and vice versa. The displaying and/or the guidance for the user may comprise providing the rotation angle to the user interface, such that the respective angle around which the collimator has to be rotated is displayed and the user can arrange the apparatus, the collimator, to the optimal collimator position. On the other hand the provided rotation angle is used for guiding the user for arriving at the optimized collimator position of the apparatus without displaying the information about optimal collimation position. Moreover, also a combination of both embodiments is possible, wherein the present status of the apparatus and/or of the collimator is displayed and the apparatus comprises a guidance to the final optimal collimation position. This guidance may comprise any visual interaction with the user showing the start position and the end position of the collimator and how and in which direction the user has to move the apparatus, the collimator, and/or how the processing unit may automatically move the apparatus and/or the collimator to the and position, which is the optimized collimation position. Further, the user interface may be configured to receive an input from the user for providing further information to the processing unit and/or for making amendments or adjustments to the optimal collimation position.

According to a second aspect of the present invention, an X-ray system is provided, which comprises the apparatus according to any one of the embodiments described herein. The system may further comprise an X-ray source emitting electromagnetic radiation, and the collimator of the apparatus for collimating the electromagnetic radiation emitted from the X-ray source. Further, the system may comprise a detector for detecting the emitted and collimated electromagnetic radiation. In particular the X-ray system may be configured to perform the method as described with a further aspect of the present invention.

According to a third aspect of the present invention, a method for moving a collimator for collimate electromagnetic radiation emitted from an X-ray source is provided. The method comprises the steps of acquiring data of an anatomy of a patient using a sensor, calculating an optimal collimation position of a collimator based on the acquired data using a processing unit, determining a movement of the collimator based on the calculated optimal collimation position using a processing unit, wherein the movement of the collimator comprises a rotation of the collimator around a central axis of the collimator. The method may further be configured to perform method steps which are able to calculate and control the apparatus and its features as described herein with the other embodiments. In particular the method may comprise steps of controlling the collimator based on the calculated optimal collimation, calculating a rotation angle for rotating the collimator around the central axis of the collimator, controlling and/or moving the moving unit, which moving unit is configured for rotating the collimator around the central axis of the collimator.

According to a further aspect of the present invention, a computer program or a computer program element is provided that is characterized by being adapted to execute the method steps of the method according to one of the preceding embodiments, on an appropriate system. The computer program may comprise instructions, which, when the program is executed by a computer, cause the computer to carry out the method of any one of the embodiments as described herein above. The computer program element might therefore be stored on a computer unit, which might also be part of an embodiment of the present invention. This computing unit may be adapted to perform or induce a performing of the steps of the method described above. The computing unit can be adapted to operate automatically and/or to execute the orders of a user. A computer program may be loaded into a working memory of a data processor. The data processor may thus be equipped to carry out the method of the invention. This exemplary embodiment of the invention covers both, a computer program that right from the beginning uses the invention and a computer program that by means of an up-date turns an existing program into a program that uses the invention. Further on, the computer program element might be able to provide all necessary steps to fulfill the procedure of an exemplary embodiment of the method as described above.

According to a further aspect of the present invention, a computer readable medium, such as a CD-ROM, is presented wherein the computer readable medium has a computer program element stored on it which computer program element is described by the preceding section. A computer program may be stored and/ or distributed on a suitable medium, such as an optical storage medium or a solid-state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. The computer-readable medium may comprise instructions which, when executed by a computer, cause the computer to carry out the method of any one of the embodiments as described herein.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

### BRIEF DESCRIPTION OF THE DRAWINGS

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.
Fig. 1 shows a collimator according to an exemplary embodiment of the state of the art.
Fig. 2 illustrates a collimator according to an exemplary embodiment of the invention.
Fig. 3 illustrates a collimation area without optimal collimation.
Fig. 4 illustrates a collimation area with optimal collimation according to an exemplary embodiment of the invention.
Fig. 5 illustrates a side view of an apparatus comprising a collimator according to an exemplary embodiment of the invention.
Fig. 6 illustrates a bottom view of an apparatus comprising a collimator according to an exemplary embodiment of the invention.
Fig. 7 illustrates a flow chart illustrating a method according to an exemplary embodiment of the invention.

### DETAILED DESCRIPTION OF EMBODIMENTS

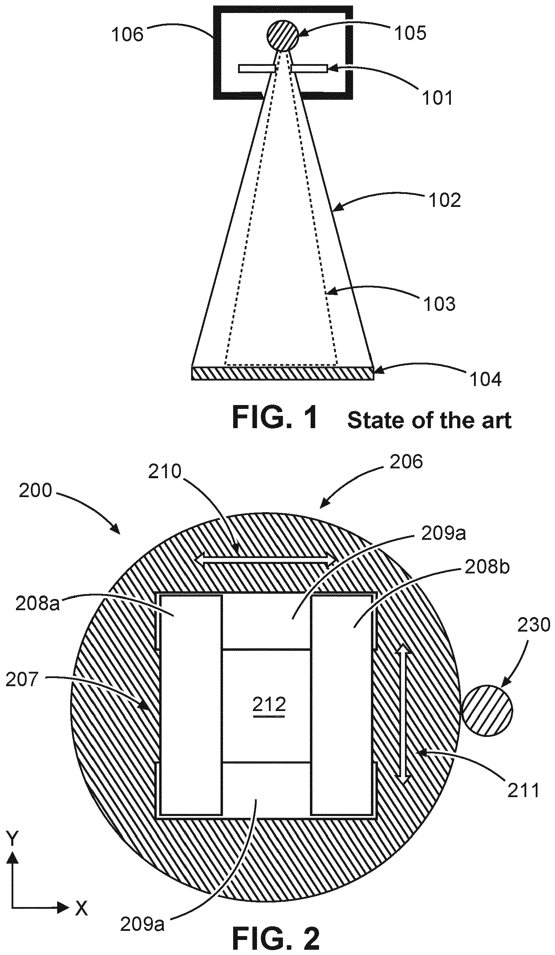

In Fig. 1 there is shown a collimator 101 according to an exemplary embodiment of the state of the art. The collimator 101 is arranged together with an X-ray source 105 inside a housing 106. The X-ray source 105 emits electromagnetic radiation 103, wherein this field of X-ray 103 is collimated, hence restricted in his emitted area by the collimator 101. Further in Fig. 1 a detector 104 is illustrated showing the possible field-of-view 102. Depending on the anatomy to be examined the field of view 102 and the field of the X-ray 103 differ due to the collimation performed by the collimator 101. The collimator 101 may be able to adjust the field of X-ray 103 by moving the, for example two parts of the collimator as illustrated in Fig. 1.

In Fig. 2 there is shown an apparatus 200 with a collimator 207 according to an exemplary embodiment of the invention. The apparatus 200 of Fig. 2 is illustrated in a schematically manner, wherein some elements of the apparatus 200 may be omitted. The collimator 207 is configured for collimating electromagnetic radiation emitted from an X-ray source, wherein the electromagnetic radiation will be emitted through the collimator opening 212 onto the anatomy of a patient. For collimating the collimator 207 may be able to adjust the size of the collimator opening 212 by moving its collimator parts. A sensor (not illustrated in Fig. 2) may be configured for acquiring data of the anatomy of a patient. The processing unit is according to an exemplary embodiment configured for calculating an optimal collimation position of the collimator 207 based on the acquired data of the sensor. Further, the processing unit may be configured to determine a movement of the collimator 207 based on the calculated optimal collimation position. The movement of the collimator 207 may comprise a rotation of the collimator 207 around a central axis of the collimator 207. This central axis of the collimator 207 as illustrated in Fig. 2 extends perpendicular to the plane formed by the X axis and the Y axis. In other words when viewed from below as illustrated with Fig. 2 the collimator 207 would rotate along the plane formed by the X axis and the Y axis. According to a further embodiment the apparatus 200 may comprise a moving unit 230 for moving the collimator 207. The apparatus 200 may comprise, according to a further embodiment, a rotatable mounting element 206, wherein the collimator 207 is attached to the rotatable mounting element 206, which is configured for rotating the collimator 207 around the central axis of the collimator 207. The mounting element 206 may be rotatable by the moving unit 230. The processing unit may further configured to control the moving unit 230 depending on the calculated optimal collimation position for rotating the collimator 207.

As illustrated in Fig. 2, the collimator 207 according to this exemplary embodiment comprises a plurality of movable collimator plates 208 a, b and 209 a, b. The collimator plates 208 a, b and 209 a, b are configured for being moved along a first direction 210 and/or along the second direction 211 for forming a collimation area. In particular the collimator plates 208 a, b and 209 a, b are forming the size of the collimator opening 212. For instance the collimator plates 208 a, b and 209 a, b may be arranged in pairs, wherein the pairs are configured to move together in a synchronous manner into the direction to the center and/or into the direction to the outside. In other words, one pair 208 a, b is able to move in a synchronous manner into the direction of the center of the collimator and/ or to the other opposite side, the outside and the other collimator plate pair 209 a, b may also be able to move in this manner, independently from the other plate pair. The first pair may be the collimator to plates 208 a, b and the second pair may be to collimator plates 209 a, b, wherein the first pair may be movable along the X axis direction 210, which is the first direction and the first pair may be movable along the Y-axis direction 211 which is the second direction. The same applies to the second pair of the collimator plates. The collimator plates 208 a, b and 209 a, b may be movable in the symmetrical manner which means that the plates of one pair are moved about the same distance in a synchronous manner into the direction of the center and/or into the direction of the outside. On the other hand the collimator plates 208 a, b and 209 a, b may be movable in an asymmetrical manner, which means that the plates of one pair may be moved about different distances into the same or different directions.

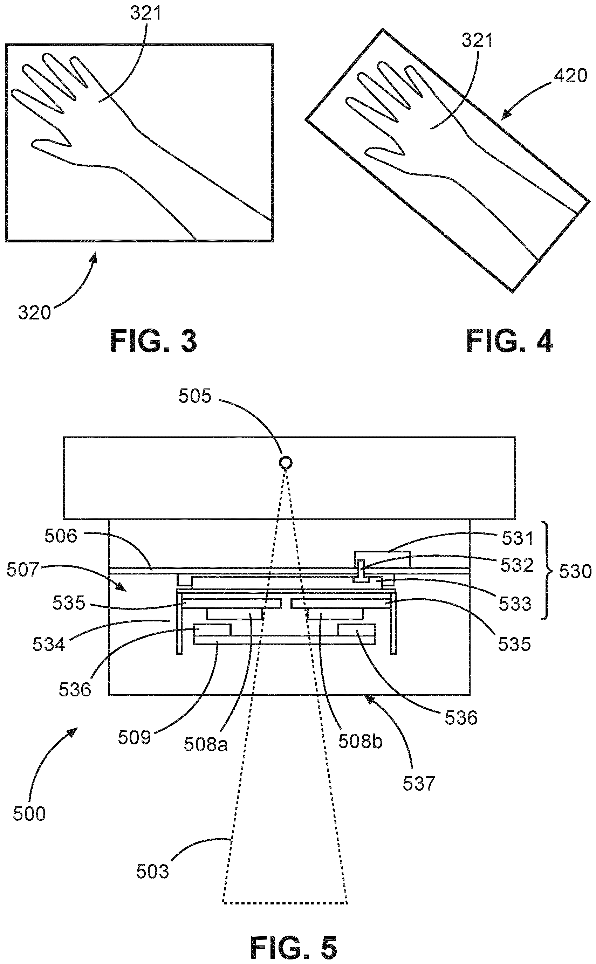

The rotation of the collimator 207 may be performed along the plate formed by the X axis and the Y axis, wherein the collimator 207 may rotate together with the mounting element 206. When the collimator 207 may be moved, rotated, also the collimator plates 208, 209 are rotated which results in the rotated collimation area as illustrated in Fig. 4.

In Fig. 3 there is shown a collimation area 320 without optimal collimation. An anatomy of a patient 321 is illustrated wherein the collimation area 320 is applied by the collimated collimator 207, 507 to the anatomy of the patient 321 without the movement of the collimator 207, 507 to the optimal collimation point. This means that a large area is irradiated by the electromagnetic radiation emitted from the X-ray source, wherein the irradiated area in this case corresponds to the collimation area 320. As can be seen in comparison with Fig. 4 the collimation area 320 is larger than the collimation area 420 in Fig. 4.

In Fig. 4 there is shown a collimation area 420 with optimal collimation according to an exemplary embodiment of the invention. As can be seen the collimation area 420 in comparison to the collimation area 320 from Fig. 3 is rotated and the size of the collimation area 420 is smaller than the size of the collimation area 320 shown in Fig. 3. Therefore the irritated area is smaller when the collimator 207 is rotated to the optimal collimator position.

The processing unit may be configured to determine anatomical landmarks from the acquired data. According to the illustration of Fig. 3 or 4 the processing unit may be configured to determine the anatomical landmarks from an image acquired by the sensor wherein the image according to Fig. 3 or 4 may be an image of the hand of the patient. The determining of anatomical landmarks by the processing unit may be independent from the collimation area 320, 420, in particular it may be independent whether the collimator 207 is arranged at the optimal collimator position because the determining of anatomical landmarks may be performed before the collimator 207 is moved/arranged at the optimized collimator position. Afterwards, the optimal collimation is calculated based/using the determined anatomical landmarks.

In Fig. 5 there is shown a side view of an apparatus 500 comprising a collimator 507 according to an exemplary embodiment of the invention. The apparatus 500 of Fig. 5 is an exemplary embodiment with an exemplary moving unit 530, wherein the moving unit 530 is not limited to this embodiment. Also other moving units 530 comprising different elements may be applicable, which are suitable for moving the collimator 507. The apparatus 500 further comprises a housing 537, wherein the collimator 507 is arranged inside the housing 537 and wherein the collimator 507 is rotatable inside the housing 537. The collimator 507 comprises collimator plates 508a, b extending along the first direction and collimator plates 509, wherein only one plate of this collimator plate pair 509 is visible and this pair extends along a perpendicular direction than the first plate pair 508. The moving unit 530 according to this embodiment comprises a motor 531 arranged at the mounting element 506 wherein the motor is configured to move the collimator 507. The movement of the motor 531 is transferred by a shaft 532 to a slewing ring 533, wherein the slewing ring 533 is arranged and/or attached to the collimator 507 by a mounting plate 534. The slewing ring 533 may comprise two parts an inner part and an outer part wherein the inner part is moved together with the collimator 507. The shaft 532 transfers the force of the motor 531 to the inner part and moves the collimator 507. According to this configuration a rotation of the collimator 507 may be realized additionally to the movement of the collimator plates 508 and 509 along the first and second directions. For moving the collimator plates 508 and 509 the collimator 507 may comprise actuators 535, 536 for each collimator plate 508a, b and 509 (a. b) by which the collimator plates 508 and 509 can be moved along the first and/or the second direction.

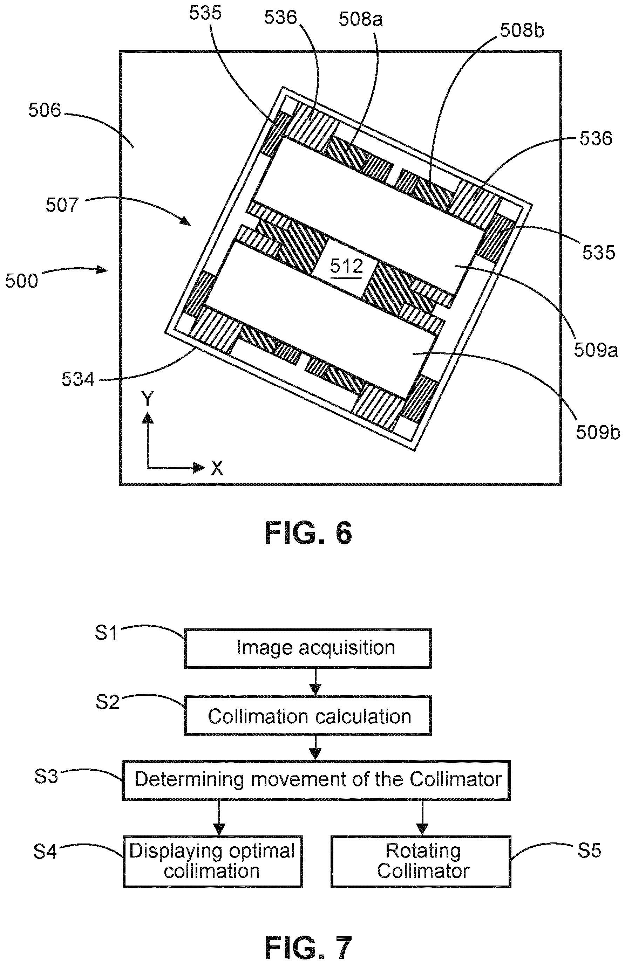

In Fig. 6 there is shown a bottom view of the apparatus 500 comprising a collimator according to an exemplary embodiment of the invention as described with Fig. 5. In this figure the collimator 507 is arranged in a rotated position, which may be the optimal collimator position. The collimator 507 comprises the same elements and features as the collimator 507 described with Fig. 5, wherein now all collimator plates 508 a, b and 509 a, b are visible from below. The size of the collimator opening 512 depends on the arrangements of the four collimator plates 508 and 509 and accordingly also the collimation area. As can be seen in Fig. 6 only the collimator 507 is moved, i.e. rotated, and the mounting element 506 is not moved, i.e. remains at its position.

In Fig. 7 there is shown a flow chart illustrating a method according to an exemplary embodiment of the invention. The flow chart illustrates different steps of the method for collimating electromagnetic radiation emitted from an X-ray source, wherein the order of the steps is not limited, which means the order of the steps may be changed or some steps may be omitted or further steps may be added. The method comprises the first step S1 acquiring data of an anatomy of a patient using a sensor. The method comprises the step S2 calculating an optimal collimation position of the collimator based on the acquired data using a processing unit. Further the method comprises the step S3 determining a movement of the collimator based on the calculated optimal collimation position using a processing unit, wherein the movement of the collimator comprises a rotation of the collimator around a central axis of the collimator. In Fig. 7 the method may comprise additional steps S4 and S5, which are displaying an optimal collimation by a user interface to the user and in step S5 the processing unit may rotate the collimator to calculated optimal collimator position. Further steps which the method may carry out and which may be performed by the processing unit may be calculating a first movement of the collimator along a first direction, calculating a second movement of the collimator along a second direction, wherein the first direction and the second direction are perpendicular to each other. Furthermore the method may comprise the step of controlling the optimal collimation position of the collimator in the first direction and/or in the second direction using the processing unit, wherein the collimator is moved to the optimal collimation position based on at least one of the calculated first movement, second movement, or the rotation of the collimator. Moreover further additional steps of the method may be calculating the rotation of the collimator simultaneously with at least one of the calculation of the movement of the collimator along the first direction and/or the calculation of the movement of the collimator along the second direction.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 101: collimator
- 102: field of view
- 103, 503: field of X-ray
- 104: detector
- 105, 505: X-ray source
- 106: Housing
- 200,500: apparatus
- 206: mounting element
- 207, 507: collimator
- 208a, b: collimator plate
- 209a, b: collimator plate
- 210: first direction
- 211: second direction
- 212, 512: collimator opening
- 230: moving unit
- 320, 420: collimation area
- 321: anatomy of patient
- 508a, b: collimator plate
- 509: collimator plate
- 530: moving unit
- 531: motor
- 532: shaft
- 533: slewing ring
- 534: mounting plate
- 535, 536: actuator for collimator plate
- 537: housing

## Claims

1. An apparatus, comprising
a collimator configured for collimate electromagnetic radiation emitted from an X-ray source,
a sensor configured for acquiring data of an anatomy of a patient,
a processing unit configured to
calculate an optimal collimation position of the collimator based on the acquired data of the sensor,
determine a movement of the collimator based on the calculated optimal collimation position,
wherein the movement of the collimator comprises a rotation of the collimator around a central axis of the collimator.

2. The apparatus according to claim 1, wherein the processing unit is configured to control the collimator based on the calculated optimal collimation.

3. The apparatus according to claim 1 or 2, wherein the calculation of the optimal collimation position comprises calculating a rotation angle for rotating the collimator around the central axis of the collimator.

4. The apparatus according to any one of the preceding claims, further comprising
a rotatable mounting element,
wherein the collimator is attached to the rotatable mounting element, which is configured for rotating the collimator around the central axis of the collimator,
a moving unit, wherein the mounting element is rotatable by the moving unit.

5. The apparatus according to claim 4, wherein the processing unit is further configured to control the moving unit depending on the calculated optimal collimation position for rotating the collimator.

6. The apparatus according to any one of the preceding claims wherein the processing unit is further configured to
calculate first movement of the collimator along a first direction,
calculate a second movement of the collimator along a second direction,
wherein the first direction and the second direction are perpendicular to each other.

7. The apparatus according to claim 6,
wherein the processing unit is further configured to control the optimal collimation position of the collimator in the first direction and/or in the second direction,
wherein the collimator is moved to the optimal collimation position based on at least one of the calculated first movement, second movement, or the rotation of the collimator.

8. The apparatus according to any one of the preceding claims 6 or 7, wherein the processing unit is configured to calculate the rotation of the collimator simultaneously with at least one of the calculation of the movement of the collimator along the first direction or the calculation of the movement of the collimator along the second direction.

9. The apparatus according to any one of the preceding claims, wherein the collimator comprises movable collimator plates configured for being moved along the first direction and/or along the second direction for forming a collimation area,
wherein the collimator comprises in particular at least four movable collimator plates.

10. The apparatus according to any one of the preceding claims, wherein the apparatus further comprises a housing, wherein the collimator is arranged inside the housing and wherein the collimator is rotatable inside the housing.

11. The apparatus according to any one of the preceding claims,
wherein the processing unit is further configured to determine anatomical landmarks from the acquired data,
wherein the optimal collimation is calculated based/using the determined anatomical landmarks.

12. The apparatus according to any one of the preceding claims,
wherein the processing unit is further configured to calculate the optimal collimation based on at least one of a trained neural network using end-to end learning, or deep learning.

13. The apparatus according to any one of the preceding claims, wherein the apparatus further comprises a user interface configured to display to a user the calculated optimal collimation and/or wherein the processor is further configured to provide a guidance to the user for receiving optimal collimation.

14. An X-ray system comprising the apparatus according to an of the preceding claims, the X-ray system further comprising
an X-ray source emitting electromagnetic radiation,
the collimator of the apparatus for collimating the electromagnetic radiation emitted from the X-ray source.

15. Method for moving a collimator for collimate electromagnetic radiation emitted from an X-ray source, the method comprising,
acquiring data of an anatomy of a patient using a sensor,
calculating an optimal collimation position of a collimator based on the acquired data using a processing unit,
determining a movement of the collimator based on the calculated optimal collimation position using the processing unit,
wherein the movement of the collimator comprises a rotation of the collimator around a central axis of the collimator.
